# EUROPEAN PATENT SPECIFICATION

(11) **EP 4 237 057 B1**
(45) Date of publication and mention of the grant of the patent: **30.04.2025**
(21) Application number: 21807220.5
(22) Date of filing: 27.10.2021
(51) Int. Cl.: A61M 21/02

(54) **SLEEP AID APPARATUS**
SCHLAFHILFEVORRICHTUNG
APPAREIL D'AIDE AU SOMMEIL

(30) Priority: 30.10.2020 GB 202017234
(43) Date of publication of application: 06.09.2023
(73) Proprietor: Mayborn (UK) Limited, Newcastle upon Tyne, Tyne and Wear NE12 8EW (GB)
(72) Inventor: HUME, Josh, Newcastle upon Tyne, Tyne and Wear NE12 8EW (GB); HESELWOOD, Gerard, Newcastle upon Tyne, Tyne and Wear NE12 8EW (GB); ALFONSO-MILLER, Pam, Newcastle upon Tyne, Tyne and Wear NE12 8EW (GB); ELLIS, Jason, Newcastle upon Tyne, Tyne and Wear NE12 8EW (GB)
(74) Representative: Bloor, Sam
(86) International application number: PCT/GB2021/052790
(87) International publication number: WO 2022/090713

(56) References cited:
- EP-A1- 3 297 218
- WO-A1-2012/011869
- WO-A1-2019/212901
- AU-A4- 2019 100 480
- US-A1- 2017 065 792
- US-A1- 2017 189 640
- US-B1- 6 236 622
- US-B2- 10 376 670
- US-B2- 7 722 212

## Description

The present invention relates to a sleep aid apparatus, in particular, a sleep aid apparatus for use in settling infants to sleep.

### Background

It is a common concern to get the best, least disturbed sleep possible. In particular, parents and carers of infants often worry about whether their child is sleeping properly.

Many of the aspects of infant sleep that distress parents and carers are actually developmentally normal. Compared to adults and older children, infants have shorter sleep cycles, lighter sleep and different types of sleep within a particular sleep cycle. Newborn infants can spend up to 70% of their time sleeping and don't have a mature circadian rhythm. Their sleep is distributed across both day and night, and their sleep-wake patterns are not regulated by melatonin, a hormone secreted by the pineal gland, that rises significantly at night (dark), peaking overnight, becoming barely detectable during the day. At this early age, infant sleep-wake patterns are more related to hunger, and their comfort needs, but they can be influenced by the environment they are in.

At birth, infants cannot see, because response to information from the rods and cones, (which mediate visual function) are not fully developed. However, infants are sensitive to light due to the presence of specialized cells in the retina called intrinsically photosensitive retinal ganglion cells (ipRGCs). These cells play no part in visual function but have active connections with the suprachiasmatic nucleus: the principal circadian pacemaker in the brain, and also project to the limbic region, affecting cognition and mood. This means that babies can be affected by exposure to light even before they can "see" it.

As infants continue to mature they fall asleep faster, sleep less during the day and have longer sustained sleep periods. Melatonin begins to regulate the infant sleep cycle. At this stage they are capable of learning how to self-soothe after a night-time awakening. Infants who self-soothing are able to settle themselves to sleep without needing intervention from a carer.

Light is a strong regulator of the circadian cycle. Exposure to light during the night can impact circadian rhythms by suppressing melatonin. Melatonin suppression is mediated by light intensity, exposure, and wavelength.

Known sleep aid apparatus provide images and sound as distraction to encourage the user to fall asleep. Such devices provide multiple light and colour settings, multiple projected images and patterns, as well as several sound files. Sound files sometimes include a "white noise" option.

One of the drawbacks of the solutions according to the prior art is that images and sound that they emit stimulate or excite infants. Such sleep aid apparatuses are also used with infants who awaken during the night. However, this provides further stimulation at an inappropriate time, disturbing an infant so that he or she needs intervention by a carer. A further drawback of the prior art is that white noise is an artificial noise that is unrepresentative of naturally occurring sounds meaning that while white noise may be distracting it is not calming for the user nor for others overhearing it.

Accordingly, it is an object of the invention to provide an apparatus which calms an infant. In particular, the apparatus creates an environment for an infant that is conducive for sleep without distracting others. A further object of the invention is to provide apparatus which is conducive for self-soothing, so that an infant does not feel upset during a night-time awakening and does not require carer intervention to settle back to sleep. A yet further object of the invention is to provide an apparatus which does not interfere with an infant's developing circadian rhythm. Another object of the present invention is to provide an apparatus which does not disrupt the melatonin suppression of a carer.

### Summary of the Invention

According to a first aspect of the invention, there is provided a sleep aid apparatus including:
a light source adapted to generate a light signal, projected from said sleep aid apparatus with a first luminosity, wherein said first luminosity is periodically variable and wherein said light signal has a wavelength selected from a range of 600nm to 700nm; and
a sound source adapted to generate an audio signal, wherein within a predetermined frequency range said audio signal has a signal intensity which decreases as the frequency increases.

Aptly, within said predetermined frequency range said signal intensity may be inversely proportional to frequency. In this way, the apparatus generates an audio signal of pink noise, a category of noise which is commonly found in biosystems such as the firing of a neuron, a heartbeat, or the noise of the ocean. The audio signal may be a low frequency sound as is predominant in the womb environment. The audio signal is thus soothing and balanced to the human ear without sounding overly bright or artificial.

Aptly, within said predetermined frequency range said signal intensity may be inversely proportional to the square of the frequency. In this way, the apparatus generates an audio signal of Brownian noise which further reduces the proportion of high frequency sound in the audio signal.

Aptly, the volume of said audio signal may be limited to at least one of 80 dBA time averaged emission or 110 dBC peak emission, at a distance of 500 mm. In this way, the audio signal is appropriate for an infant's developing hearing.

Aptly, said audio signal may be attenuated for frequencies above a predetermined frequency. Aptly, said audio signal may be attenuated for frequencies above 1kHz. In these ways, the audio signal is adapted to mask high frequencies from infants. Compared to the adult ear canal, the infant ear canal is smaller and more resonant, amplifying higher frequencies. An attenuated audio signal is thus protective to infant hearing.

Aptly, said sound source may be adapted to generate said audio signal for a first predetermined time period. Aptly, said first predetermined period may be 40 minutes. In this way, the apparatus is automatically turned off without user intervention.

Aptly, said audio signal may be continuous. In this way, the audio signal is emitted without interruption that may cause distraction.

Aptly, the apparatus may further comprise an audio sensor for detecting an external audio signal. Aptly, said audio signal may be generated in response to the detected external audio signal. In this way, the apparatus activates without user intervention. An infant will thus learn to self soothe should they awaken.

Aptly, the apparatus may further comprise a further light source adapted to selectively generate a further light signal, projected from said sleep aid apparatus with a second luminosity and wherein said further light signal has a wavelength selected from a range of 600nm to 700nm. Aptly the further light signal is constant. Aptly, said first luminosity or said second luminosity may be selectively limited to a maximum of 200 Lux. In these ways, the apparatus provides a background light with lower risk of interrupting the circadian rhythm of a user, such as an infant or a carer. The wavelength of the light reduces the risk of disrupting a user's melatonin levels.

Aptly the sleep aid apparatus further comprises a diffuser element configured to evenly diffuse the further light signal.

Aptly, said first luminosity periodically may vary at a predetermined rate. Aptly, said predetermined rate may be within a range of 6 times per minute to 40 times per minute. More aptly, said predetermined rate may be within a range 8 times per minute to 20 times per minute. Even more aptly, said predetermined rate may be configured to incrementally change over a second predetermined time period. Aptly, said first luminosity periodically may vary between a maximum luminosity and minimum luminosity, and wherein said minimum luminosity is 5% of said maximum luminosity.

In these ways, the first light signal is adapted to pulse and at a rate matching the user's breathing rate. The matched pulsing provides a relaxing calm environment that is conducive to sleep. Slow breathing can have beneficial physiological effects, including but not limited to: muscle relaxation and lowered blood pressure. Matching breathing pattern to the pulsing lights on this setting helps users relax and primes the body for sleep. In particular, the apparatus pulses at rate that incrementally slows thereby encouraging the user's breathing to slow concurrently or synchronously.

Aptly the sleep aid apparatus is configured to project the first light signal in a narrow beam. Further, the narrow beam projects from the apparatus in a conical configuration such that the diameter of the narrow beam is from 30cm to 60cm at a distance of 140cm from the apparatus.

### Brief Description of the Drawings

Embodiments of the invention are now described, by way of example only, hereinafter with reference to the accompanying drawings, in which:
- **Figure 1.**: shows a side view of an example sleep aid apparatus according to the invention;
- **Figure 2.**: shows an upper perspective view of the example of Figure 1;
- **Figure 3.**: shows a lower perspective view of the example of Figure 1;
- **Figure 4.**: shows an options chart of the user interface of the sleep aid apparatus of Figure 1; and
- **Figure 5.**: shows a flow diagram for the method of operating the sleep aid apparatus of Figure 1.

In the drawings, like reference numerals refer to like parts.

### Detailed Description

Certain terminology is used in the following description for convenience only and is not limiting. For example, unless otherwise specified, the use of ordinal adjectives, such as, 'first', 'second', 'third' etc. merely indicate that different instances of like objects are being referred to and are not intended to imply that the objects so described must be in a given sequence, either temporally, spatially, in ranking or in any other manner.

Throughout the description reference is made to a "user". This term is intended to encompass both an infant and/or a carer. The context of whether the user is an infant who uses the apparatus to aid sleep or a carer, using the apparatus to alter settings etc will be readily apparent from the context of the description.

The present invention provides a sleep aid apparatus which includes both audio and visual signals to improve the sleep of an infant.

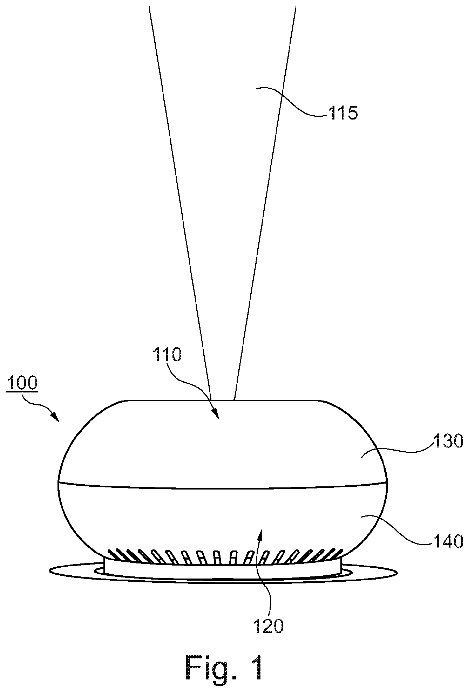

Referring to Figure 1, there is shown a side view of a sleep aid apparatus 100 including a light source 110 generating a first light signal 115 and a sound source 120 generating an audio signal.

The first light signal 115 is projected from the sleep aid apparatus 100 with a first luminosity which periodically varies. In other words the first luminosity of the first light signal changes value over time such that the luminosity moves between a minimum and maximum value. The change from the minimum to maximum value may be gradual so as to dim and brighten over an extended time period. In this way, the first light signal 115 may have a pulsing appearance, and may be referred to as a pulsed light signal.

The first luminosity may periodically vary at a predetermined rate. For example, the first luminosity may vary at a predetermined rate within the range 6 times per minute to 40 times per minute. In other words, a pulse rate of the first luminosity of the first light signal may be from 6 times per minute to 40 times per minute. A single "pulse" may be defined as a change in the first luminosity from the minimum value to the maximum value, or from the maximum value to the minimum value. In some examples, the light source may be configured to incrementally slow the periodic variation, or pulse rate. That is the pulse rate of the first luminosity may reduce over time. This can encourage the infant's breathing to slow concurrently or synchronously.

The first light signal has a wavelength selected from the range of 600nm to 700nm. Aptly the wavelength signal is in the range of 640nm to 670nm and more aptly 655nm to 660nm. The first light signal is therefore within the red part of the spectrum which does not affect the melatonin suppression of an infant when compared with blue light. The first light signal 115 is therefore less disruptive to an infant's development of a circadian rhythm. The use of red light may additionally help an infant fall asleep faster, sleep longer through the night, and may be minimally stimulating during night time awakenings, making re-settling easier.

Human perception of sound has a logarithmic response to the pressure level of sound. Within a predetermined frequency range, the sleep aid may produce an audio signal which has a signal intensity which generally decreases as the frequency increases. Within this predetermined frequency range, the audio signal may have a signal intensity which is inversely proportional to frequency. In some examples the audio signal may have a signal intensity which is inversely proportional to the square of the frequency. In other words, the audio signal may be pink noise.

Pink noise sounds more balanced and natural compared to white noise, which has constant energy across the frequency spectrum making it sound bright to human ears. The pink noise may encompass any of the frequencies that are audible to humans (20 hertz to 20,000 hertz). However the power per hertz in pink noise decreases as the frequency increases, meaning the higher frequency elements are less powerful and quieter. Through use of pink noise, the audio signal may imitate the sounds an infant is exposed to when in the womb.

In some examples the sleep aid may be configured such that the audio signal may be attenuated for frequencies above 1kHz. For example, the audio signal may have lowpass filtering applied.

The audio signal may be generated for a predetermined time period. In this way the audio signal does not require a "switch off". The audio signal may be generated for 20 minutes to 60 minutes. Aptly the audio signal may be generated for 40 minutes. 20 minutes of generated audio signal is generally sufficient to calm an infant, therefore extending the time period beyond this value increases the likelihood of allowing an infant to return to sleep.

The sleep aid apparatus 100 may include a memory. The audio signal may be stored within the sleep aid apparatus 100 as a recording or plurality of recordings, for example a sound clip. In other examples the sleep aid may include a means for generating sound. In this example the sound clips may be a set period in length, for example 10 minutes. The sleep aid may be adapted to continuously cycle the sound clip. The transition from the end of the sound clip to the start may be a smooth and/or continuous transition. In this way the audio signal may sound continuous so as to not disrupt an infant's sleep.

The sleep aid apparatus 100 may generate a further light signal. That is, an additional light signal which is visibly distinct from the first light signal. The further light signal may be projected from the sleep aid apparatus 100 with a second luminosity. In this example the second luminosity is constant. The further light signal has a wavelength selected from the range of 600nm to 700nm. Aptly the wavelength of the second light signal is in the range of 640nm to 670nm and more aptly 655nm to 660nm.

The sleep aid apparatus 100 shown in Figure 1 is includes a housing including an first portion 130 mounted to a second portion 140. The first portion may be releasably mounted to the second portion to allow opening of the sleep aid apparatus 100. In other examples the first portion 130 and second portion 140 may be one integral piece. The light source 110, sound source 120 and associated control features can be mounted within the casing. Further, an audio detector (for example a microphone) may be mounted within the casing. The audio detector may aptly be attuned to detect a "cry" noise.

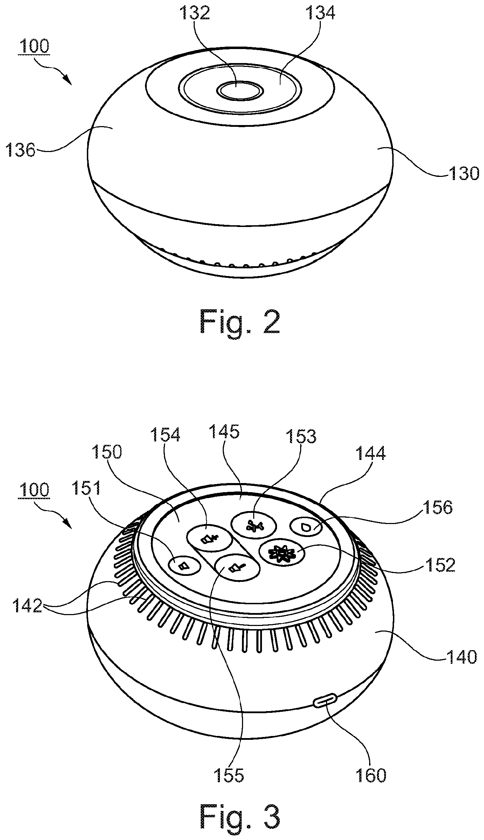

Referring now to Figure 2, the first portion 130 of the housing includes a diffuser element 136. For example, the exterior of the first portion 130 is at least partially covered by the diffuser element 136, or the diffuser element 136 may be integral with the first portion 130 of the housing. In other examples, the diffuser element 136 may be integral with the second portion 140 or be integral with both the first and second portions 130, 140.

The diffuser element 136 is translucent and arranged to allow the second light signal to project from the sleep aid apparatus 100. In other words a light source (not shown) may be arranged around the periphery of the sleep aid apparatus 100 and encapsulated by the diffuser element 136. For example, the light source may include a plurality of LEDs dispersed around the housing. Aptly the dispersion of LEDs around the housing may be substantially even.

The diffuser element 136 may disperse the second light signal evenly around the sleep aid apparatus 100. In this way, areas of inconsistently high or low luminosity compared with the rest of the housing are reduced. The diffuser element 136 may thus enable the second light signal to provide a background lighting effect. In other words, the diffuser element 136 may allow the sleep aid apparatus to emit a soft glow, where the wavelengths of light fall within the red part of the spectrum.

In this example the diffuser element 136 is a frosted material. Aptly the diffuser may be a polycarbonate material. The diffuser element 136 may reduce the volume of light able to pass therethrough. That is the diffuser element 136 may mute the light from the light source so as to reduce glare.

The sleep aid apparatus 100 may include a cover portion 134. In this example the cover portion is provided substantially centrally on the first portion 130 of the housing. The diffuser element 136 may aptly surround the cover portion 134. The cover portion 134 is adapted to act as a power switch for the sleep aid apparatus 100. In the example shown, the cover portion 134 is provided as a capacitive touch button, which is described in more detail below.

The sleep aid apparatus 100 may further include a lens 132. A light source 110 adapted to generate the first light signal 115 may be mounted within the housing such that the first light signal may be projected through the lens 132. The light source 110 may be at least one LED. In this example, the lens 132 is translucent and is arranged on the first portion 130 to allow the first light signal 115 to project from the sleep aid apparatus 100, as shown in Figure 1. Aptly, the lens 132 is adapted to project the first light signal 115 in a narrow beam. The narrow beam may be substantially conical in shape. In this example, the diameter of the narrow beam is from 30cm to 60cm, and aptly 40cm to 50cm, at a distance 140cm from the sleep aid apparatus 100. The narrow beam of the first light signal and diffused glow of the second light signal may be visibly distinct from one another.

In this example the lens 132 is positioned at the centre of the first portion 130, such that in normal use the lens 132 is at the centre on the top of the first portion 130. The lens 132 may project the narrow beam substantially vertically from the first portion 130.

In this way, the narrow beam can be projected onto a ceiling or other surface (for example a crib covering etc.).

In this example, the lens 132 is centrally mounted with respect to the cover portion 134. In this way, the sleep aid apparatus 100 includes the diffuser element 136 surrounding the cover portion 134, which in turn surrounds the lens 132. In some examples, the first portion 130 may include a concave portion or recess at the apex, into which the cover portion 134 and lens 132 are set. The sleep aid apparatus 100 therefore has a compact appearance and the cover portion 134 and lens 132 may be better protected from external impacts.

Referring now to Figure 3, a base portion 145 of the second portion 140 of the housing is illustrated. In this example the second portion 140 includes a control panel 150 provided on the base portion 145 such that it is hidden in normal use. In this example, the base portion 145 is configured to be the portion of the sleep aid apparatus 100 which sits adjacent an external surface (for example a shelf) in normal use.

The second portion 140 may include a projecting rim 144 around the periphery of the base portion 145. The projecting rim 144 protrudes from the base portion 145, thereby forming a support surface for the second portion 140. In this way the control panel 150 may be spaced apart from an external surface by the projecting rim 144, when in normal use. The projecting rim 144 may be a non-slip material such as the thermoplastic elastomer.

The control panel 150 includes a plurality of actuatable buttons. In this example, the control panel includes a projector button 151, an ambient light button 152, a sound button 153, a pair of volume buttons 154, 155, and a cry sensor button 156. It will be appreciated that one or more of these buttons may be omitted according to the desired controllability of the sleep aid functions. Other examples may of course, include alternative or additional buttons or control features, for example a single volume dial, or a touch screen interface.

The second portion 140 of the housing may include a series of apertures 142. In this example the apertures are positioned on the side of the second portion 140. For example the apertures 142 may be arranged adjacent the projecting rim 144. The apertures 142 are adapted to allow emission of audio signals from a sound source (not shown) mounted within the casing.

The sleep aid apparatus 100 may further include a port 160. In this example, the port 160 is positioned at a joining interface between the first and second portions 130, 140, though the port may alternatively be positioned in any other region of the housing. The port 160 is adapted to provide power and data transfer between the sleep aid apparatus 100 and another device such as a power source or a computer, for example via a cable. For example, the port 160 may be a USB Type C port.

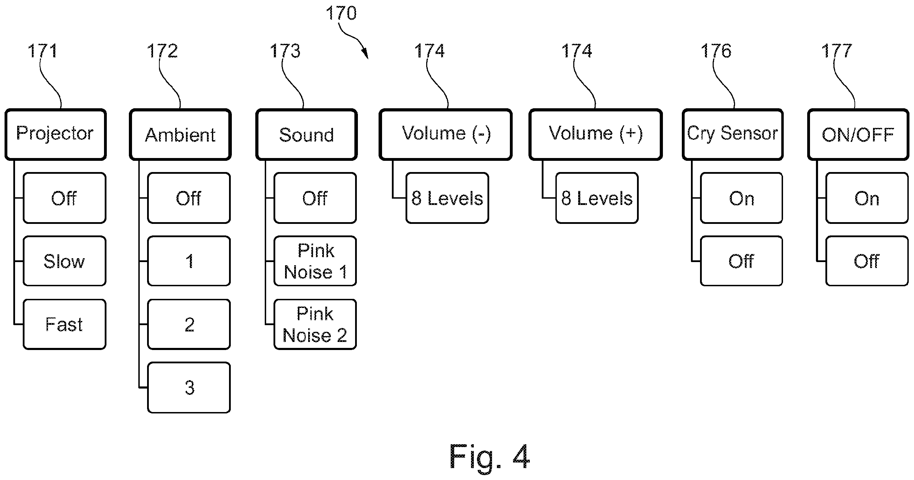

Referring now to Figure 4, there is shown the settings for an example user interface 170 for the sleep aid apparatus 100. The user interface includes the actuatable buttons of the control panel 150, as well as the power switch provided by the cover portion 134. The user interface 170 enables the user to cycle through a respective set of settings for each actuatable button 151-156 and the cover portion 134.

In this example, actuating the projector button 151 enables the user to cycle the first light signal settings 171, between 'off', 'slow' and 'fast'. When the 'off' setting is selected the first light signal is deactivated. When the 'slow' setting is selected, the luminosity of the first light signal is periodically varied at a rate of 8 cycles per minute. When the 'fast' setting is selected, the luminosity of the first light signal is periodically varied at a rate of 20 cycles per minute. Of course, other examples may include additional settings such as 'very slow' and/or 'very fast' and the periodical variation of the luminosity may be between 6 and 40 cycles per minute as appropriate. As an alternative example, any period between 6 and 40 cycles per minute may be selected via a continuously adjustable dial or "up" and "down" buttons, for example.

In this example, actuating the ambient light button 152 enables the user to cycle the second light signal settings 172 between 'off', '1', '2' and '3'. When 'off' setting is selected the second light signal is deactivated. When '1' setting is selected, the luminosity of the second light signal is set at 67 Lux. When '2' setting is selected, the luminosity of the second light signal is set at 134 Lux. When '3' setting is selected, the luminosity of the second light signal is set at 200 Lux. Other examples may include further luminosity options, from 0 Lux as a minimum and with a maximum luminosity of 200 Lux. As an alternative example, any luminosity from 0 Lux to 200 Lux may be selected via a continuously adjustable dial, or "up" and "down" buttons, for example.

In this example, actuating the sound button 153 enables the user to cycle the audio signal settings 173 for the audio signal between 'off', 'pink noise 1' and 'pink noise 2'. When 'off' setting is selected the audio signal is deactivated. When 'pink noise 1' setting is selected, a first audio signal of pink noise is emitted from the sleep aid apparatus 100. When 'pink noise 2' setting is selected, a second audio signal of pink noise with a low pass filter is emitted from the sleep aid apparatus 100. The low pass filter of the second audio signal attenuates frequencies over 1 kHz. The pink noise with a low pass filter may produce a sound which may mimic the sound spectra of a womb. Other examples may include further pink noise audio signal options.

Actuating either of the volume buttons 154, 155 enables the user to cycle the volume settings 174 for the audio signal between volume levels. In this example there are 8 volume levels. For example, actuating the first volume button 154 selectively increases the volume of the audio signal up to a maximum volume setting. Similarly, actuating the second volume button 155 selectively decreases the volume of the audio signal up to a minimum volume setting. In this example, the audio signal is set to 'pink noise 1' at the minimum volume setting as the default setting for first use or after a factory reset .

In some examples, actuating either of the volume buttons when the audio signal setting is 'off' may switche the audio signal option to 'pink noise 1' at the minimum volume setting.

Actuating the cover portion 134 may enable the user to cycle through the power state settings 177 for the sleep aid apparatus 100. When 'off' setting is selected the sleep aid apparatus 100 is in a power off state. In the power off state, the audio signal and light signals are deactivated. When 'on' setting is selected the sleep aid apparatus 100 is in a power on state. In the power on state, the sleep aid apparatus 100 generates audio and light signals according to the other settings of the user interface 170. The sleep aid apparatus 100 may include a controller configured to control the settings and communicate with the sleep aid memory.

Actuating the cry sensor button 156 enables the user to cycle the cry sensor settings 176 between 'off' and 'on'. When 'off' setting is selected the cry sensor is deactivated. When 'on' setting is selected the cry sensor is activated.

In this example regardless of whether the cry sensor setting is 'on' or 'off', if the sleep aid apparatus 100 is in a power on state then then the sleep aid apparatus 100 provides at least one of the audio signal, first light signal or second light signal.

The respective signals may be provided for a predetermined time period, thus, enabling the sleep aid apparatus 100 to be set and left. Aptly this predetermined time period may be between 20 minutes to 60 minutes and more aptly 40 minutes.

However, a user may actuate the cover portion 134 to selectively switch the sleep aid apparatus 100 into the power off state. That is, the cover portion 134 may be capable of overriding the predetermined time period the signals are active for.

In an example without a user actuation of the cover portion 134, when the sleep aid apparatus 100 reaches the end of the predetermined time period, the next action is dependent on the cry sensor setting 176. If the cry sensor setting 176 is 'off' at the end of the predetermined time period, then the respective audio or light signals are deactivated and the sleep aid apparatus 100 may switch to a power off state. If the cry sensor setting 176 is 'on' at the end of the predetermined time period, then the respective audio or light signals are deactivated and the sleep aid apparatus 100 switches to a listening state.

In the listening state, the sleep aid apparatus 100 may be configured to respond to external audio signals, for example the sound of an infant crying. For example, if an external audio signal is detected by the audio detector then the sleep aid apparatus 100 may switch to a power on state. That is, the audio and light signals at the most recent settings for a further predetermined time period may be reactivated. This reactivation may be controlled by a controller contained within the sleep aid apparatus 100 housing.

In normal use, the sleep aid apparatus 100 may be positioned so that the lens 132 and cover portion 134 face upwards. As used herein "normal use" is where the sleep aid apparatus 100 is placed on a surface and the carer does not require access to the control panel 150. The control panel 150 may be hidden from view as the sleep aid apparatus 100 rests on the surface. In this way, in normal use, a user can change the apparatus power state setting 177 by easily accessing the cover portion 134. In the example shown, the cover portion 134 may be actuated by the user tapping the cover portion 134 twice in quick succession. Thus, the power state setting 177 cannot be changed unintentionally, for example by accidentally touching the cover portion 134.

In an example with the sleep aid apparatus in a power on state and the first light signal 115 not deactivated, the first light signal 115 may project out of the casing through the lens 132. The first light signal 115 may provide a narrow beam of light extending above the sleep aid apparatus 100. The first light signal 115 has a wavelength in the range of 600nm to 700nm. The first light signal 115 varies periodically thereby providing a regularly pulsing light.

With the sleep aid apparatus 100 in a power on state and second light signal is not deactivated, the second light signal is projected through the diffuser element 136. The second light signal provides a background, or ambient, lighting effect. The second light signal has a wavelength in the range of 600nm to 700nm, for example 655nm to 660nm. The second light signal may provide an ambient light of more selectively limited luminosity than the first light signal 115.

In an example with the sleep aid apparatus 100 in a power on state and audio signal not deactivated, the audio signal may be emitted through the series of apertures 142. The audio signal setting 173 and volume setting 174 are set according by the user actuating the sound button 153 and the volume buttons 154, 155 respectively.

The carer may actuate any combination of audio signal, first light signal and second light signal including having one or more signals switched off. The large number of combination options allows for a carer to select an overall projection of signals from the sleep aid apparatus 100 that is tailored to an individual infant. Moreover, the sleep aid apparatus may save the selected settings, for example in a memory, thereby reducing subsequent set up times, and improving usability for a carer.

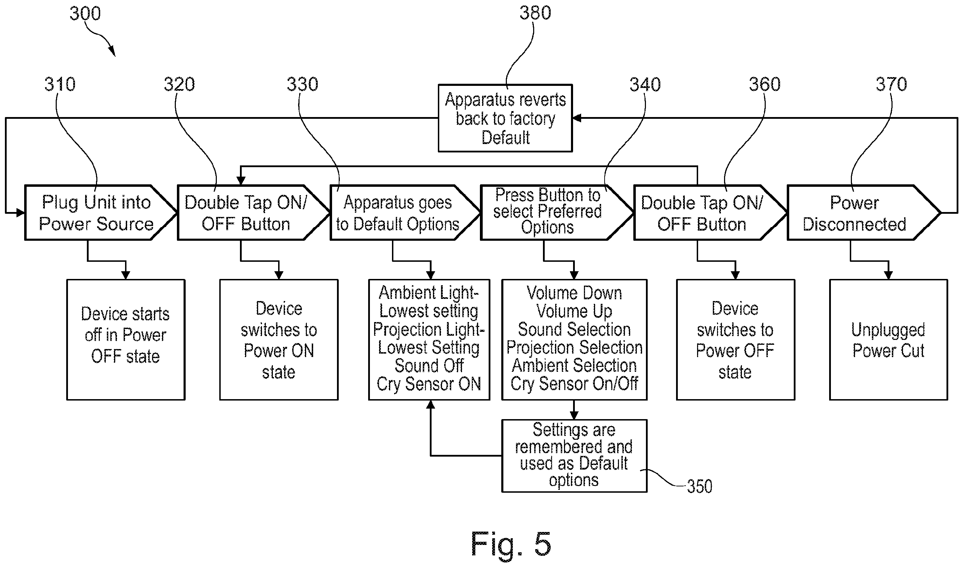

Referring now to Figure 5, there is shown an example control flow diagram 300 of the sleep aid apparatus. The control flow diagram 300 sets out an example method of operation of the sleep aid apparatus 100 in use. Initially, a power source is provided 310 to the sleep aid apparatus. In the example shown, the power source is provided via the port 160. With power source provided, the sleep aid apparatus 100 remains in the power off state.

A user actuates 320 the cover portion 134 so that the sleep aid apparatus 100 is switched to the power on state. Once switched to the power on state the sleep aid apparatus 100 is activated 330 according to its factory default settings. Specifically, in this example the first light signal setting 171 is 'slow', the second light signal setting 172 is '1', the audio signal setting 173 is 'off', and the cry sensor setting 176 is 'on'.

The user selectively actuates 340 the buttons of the control panel 150 to adjust the respective settings according to their preference. The adjusted settings are saved 350 by the sleep aid apparatus 100 as stored default settings for as long as it remains connected to the power source.

The sleep aid apparatus 100 provides audio and light signals for the duration of a predetermined period followed by switching to either the listening state or the power off state, as described above in reference to Figure 4.

If, during any predetermined period, the user actuates 360 the cover portion then the sleep aid apparatus 100 stores the most recent settings as the default settings and switches to the power off state. Should a user subsequently actuate 320 the cover portion, the sleep aid apparatus 100 reverts to a power on state using the stored default settings.

Should, at any time, the sleep aid apparatus 100 be disconnected 370 from its power source, then the stored default settings may be lost. When the power source is subsequently reconnected, the sleep aid apparatus 100 reverts 380 to the factory default settings.

Various modifications to the detailed arrangements as described above are possible.

Aspects of the user interface may be changed or removed while ensuring that the sleep aid apparatus is within the scope of the invention.

Optionally, the power source may be provided by one or more batteries mounted within the casing. The batteries may be replaceable or may be rechargeable through an electrical cable connecting through the port.

Optionally, the port additionally or alternatively provides a means for data transfer, for example using a connection through a USB cable. Optionally, the data transfer may be achieved by known wireless means, for example Bluetooth. In this way, any of the settings of the sleep aid apparatus may be controlled by an additional device, such as a computer or a smartphone app. The device may be configured to receive additional audio files to enable one or more of the audio signals to be modified or changed.

Although in the example described above the control panel includes a number of actuatable buttons, in other examples the control panel may alternatively or additionally include a touch screen control panel. The touch screen control panel may provide a user interface enabling the user to select any of the settings described above in relation to the actuatable buttons. In a further example, the control panel may be omitted and the device may be controlled wirelessly via a remote control or an app in a smart device (e.g. a portable electronic device such as a smartphone), or via voice activation for example.

Yet further examples of a sleep aid apparatus may include a ambient light sensor configured to activate the sleep aid apparatus when the ambient light drops below a predetermined threshold value. An ambient light sensor may be used in combination with or as an alternative to the control panel described above.

It will be clear to a person skilled in the art that features described in relation to any of the embodiments described above can be applicable interchangeably between the different embodiments. The embodiments described above are examples to illustrate various features of the invention.

The above described arrangement provides an audio and visual device for calming an infant. The device is further advantageous as the combination of the audio and visual signals is adjusted to infant senses such that infant circadian rhythms remain largely unaffected while providing soothing to the infant without carer input.

The above described arrangement provides the advantage that an audio signal which is soothing and balanced to the human ear may be provided whilst remaining appropriate for an infant's developing hearing. In some examples, the audio signal may imitate a womb environment.

The above described arrangement provides the advantage that activation without direct user input may be provided. This reduces the burden on a carer and may additionally aid in infant self-soothing.

The above described arrangement provides the advantage that a background light with a wavelength that reduces the risk of disrupting a user's melatonin levels can be provided. This may therefore significantly lower the risk of interrupting the circadian rhythm of a user (both carer and infant).

The reader's attention is directed to all papers and documents which are filed concurrently with or previous to this specification in connection with this application and which are open to public inspection with this specification.

The present invention is defined by the following claims.

## Claims

1. A sleep aid apparatus (100) comprising:
a light source (110) adapted to generate a light signal (115), projected from said sleep aid apparatus (100) with a first luminosity, wherein said first luminosity is periodically variable and wherein said light signal has a wavelength selected from a range of 600 nm to 700 nm; and
a sound source (120) adapted to generate an audio signal,
**characterised in that**
within a predetermined frequency range said audio signal has a signal intensity which decreases as the frequency increases.

2. A sleep aid apparatus (100) according to claim 1, wherein within said predetermined frequency range, said signal intensity is inversely proportional to frequency.

3. A sleep aid apparatus (100) according to claim 1, wherein within said predetermined frequency range said signal intensity is inversely proportional to the square of the frequency.

4. A sleep aid apparatus (100) according to any preceding claim, wherein the volume of said audio signal is limited to at least one of 80 dBA time averaged emission or 110 dBC peak emission, at a distance of 500 mm.

5. A sleep aid apparatus (100) according to any preceding claim, wherein said audio signal is attenuated for frequencies above a predetermined frequency, and wherein said audio signal is optionally attenuated for frequencies above 1kHz.

6. A sleep aid apparatus (100) according to any preceding claim, wherein said sound source (120) is adapted to generate said audio signal for a first predetermined time period, and wherein said first predetermined period is optionally 40 minutes.

7. A sleep aid apparatus (100) according to any preceding claim, wherein said audio signal is continuous.

8. A sleep aid apparatus (100) according to any preceding claim, wherein the apparatus (100) further comprises an audio sensor for detecting an external audio signal, and wherein said audio signal is optionally generated in response to the detected external audio signal.

9. A sleep aid apparatus (100) according to any preceding claim, further comprising a further light source adapted to selectively generate a further light signal, projected from said sleep aid apparatus (100) with a second luminosity and wherein said further light signal has a wavelength selected from a range of 600nm to 700nm, and wherein said second luminosity is optionally constant, and wherein the sleep aid apparatus (100) optionally further comprises a diffuser element (136) configured to evenly diffuse the further light signal.

10. A sleep aid apparatus (100) according to any preceding claim, wherein said first luminosity is selectively limited to a maximum of 200 Lux.

11. A sleep aid apparatus (100) according to any of claims 9 to 10, wherein said second luminosity is selectively limited to a maximum of 200 Lux.

12. A sleep aid apparatus (100) according to any preceding claim, wherein said first luminosity is periodically variable at a predetermined rate, and wherein said predetermined rate is optionally within a range of 6 times per minute to 40 times per minute, for example, said predetermined rate is optionally within a range 8 times per minute to 20 times per minute.

13. A sleep aid apparatus (100) according to claim 12, wherein said predetermined rate is configured to incrementally change over a second predetermined time period.

14. A sleep aid apparatus (100) according to any preceding claim, wherein said first luminosity periodically varies between a maximum luminosity and minimum luminosity, and wherein said minimum luminosity is 5% of said maximum luminosity.

15. A sleep aid apparatus (100) according to any preceding claim, wherein the apparatus (100) is configured to project the first light signal in a narrow beam, and wherein the narrow beam optionally projects from the apparatus (100) in a conical configuration such that the diameter of the narrow beam is from 30cm to 60cm at a distance of 140cm from the apparatus (100).

## Patentansprüche

1. Schlafhilfevorrichtung (100), umfassend:
eine Lichtquelle (110), die dazu ausgelegt ist, ein Lichtsignal (115) zu erzeugen, das von der Schlafhilfevorrichtung (100) mit einer ersten Leuchtkraft projiziert wird, wobei die erste Leuchtkraft periodisch variabel ist und wobei das Lichtsignal eine Wellenlänge aufweist, die aus einem Bereich von 600 nm bis 700 nm ausgewählt ist; und
eine Tonquelle (120), die dazu ausgelegt ist, ein Audiosignal zu erzeugen, **dadurch gekennzeichnet, dass** dass das Audiosignal innerhalb eines vorbestimmten Frequenzbereichs eine Signalintensität aufweist, die mit zunehmender Frequenz abnimmt.

2. Schlafhilfevorrichtung (100) nach Anspruch 1, wobei innerhalb des vorbestimmten Frequenzbereichs die Signalintensität umgekehrt proportional zur Frequenz ist.

3. Schlafhilfevorrichtung (100) nach Anspruch 1, wobei innerhalb des vorbestimmten Frequenzbereichs die Signalintensität umgekehrt proportional zum Quadrat der Frequenz ist.

4. Schlafhilfevorrichtung (100) nach einem der vorstehenden Ansprüche, wobei die Lautstärke des Audiosignals auf mindestens eines von 80 dBA zeitgemittelte Emission oder 110 dBC Spitzenemission bei einem Abstand von 500 mm begrenzt ist.

5. Schlafhilfevorrichtung (100) nach einem der vorstehenden Ansprüche, wobei das Audiosignal für Frequenzen über einer vorbestimmten Frequenz gedämpft wird und wobei das Audiosignal optional für Frequenzen über 1 kHz gedämpft wird.

6. Schlafhilfevorrichtung (100) nach einem der vorstehenden Ansprüche, wobei die Tonquelle (120) dazu ausgelegt ist, das Audiosignal für eine erste vorbestimmte Zeitspanne zu erzeugen, und wobei die erste vorbestimmte Zeitspanne optional 40 Minuten beträgt.

7. Schlafhilfevorrichtung (100) nach einem der vorstehenden Ansprüche, wobei das Audiosignal kontinuierlich ist.

8. Schlafhilfevorrichtung (100) nach einem der vorstehenden Ansprüche, wobei die Vorrichtung (100) ferner einen Audiosensor zum Erkennen eines externen Audiosignals umfasst und wobei das Audiosignal optional als Reaktion auf das erkannte externe Audiosignal erzeugt wird.

9. Schlafhilfevorrichtung (100) nach einem der vorstehenden Ansprüche, ferner umfassend eine weitere Lichtquelle, die dazu ausgelegt ist, selektiv ein weiteres Lichtsignal zu erzeugen, das von der Schlafhilfevorrichtung (100) mit einer zweiten Leuchtkraft ausgestrahlt wird, und wobei das weitere Lichtsignal eine Wellenlänge aufweist, die aus einem Bereich von 600 nm bis 700 nm ausgewählt ist, und wobei die zweite Leuchtkraft optional konstant ist, und wobei das Schlafhilfevorrichtung (100) ferner optional ein Diffusorelement (136) umfasst, das dazu konfiguriert ist, das weitere Lichtsignal gleichmäßig zu streuen.

10. Schlafhilfevorrichtung (100) nach einem der vorstehenden Ansprüche, wobei die erste Leuchtkraft selektiv auf maximal 200 Lux begrenzt ist.

11. Schlafhilfevorrichtung (100) nach einem der Ansprüche 9 bis 10, wobei die zweite Leuchtkraft selektiv auf maximal 200 Lux begrenzt ist.

12. Schlafhilfevorrichtung (100) nach einem der vorstehenden Ansprüche, wobei die erste Leuchtstärke periodisch mit einer vorbestimmten Rate variiert, und wobei die vorbestimmte Rate optional in einem Bereich von 6 Mal pro Minute bis 40 Mal pro Minute liegt, beispielsweise liegt die vorbestimmte Rate optional in einem Bereich von 8 Mal pro Minute bis 20 Mal pro Minute.

13. Schlafhilfevorrichtung (100) nach Anspruch 12, wobei die vorbestimmte Rate so konfiguriert ist, dass sie sich über eine zweite vorbestimmte Zeitspanne schrittweise ändert.

14. Schlafhilfevorrichtung (100) nach einem der vorstehenden Ansprüche, wobei die erste Leuchtkraft periodisch zwischen einer maximalen Leuchtkraft und einer minimalen Leuchtkraft variiert und wobei die minimale Leuchtkraft 5 % der maximalen Leuchtkraft beträgt.

15. Schlafhilfevorrichtung (100) nach einem der vorstehenden Ansprüche, wobei die Vorrichtung (100) so konfiguriert ist, dass sie das erste Lichtsignal in einem schmalen Strahl projiziert, und wobei der schmale Strahl optional von der Vorrichtung (100) in einer konischen Konfiguration projiziert wird, so dass der Durchmesser des schmalen Strahls in einem Abstand von 140 cm von der Vorrichtung (100) zwischen 30 cm und 60 cm beträgt.

## Revendications

1. Appareil d'aide au sommeil (100) comprenant :
une source de lumière (110) adaptée pour générer un signal lumineux (115), projeté depuis ledit appareil d'aide au sommeil (100) avec une première luminosité, dans lequel ladite première luminosité est périodiquement variable et dans lequel ledit signal lumineux a une longueur d'onde choisie dans une plage de 600 nm à 700 nm ; et
une source sonore (120) adaptée pour générer un signal audio,
**caractérisé en ce que,** dans une plage de fréquences prédéterminée, ledit signal audio a une intensité de signal qui diminue à mesure que la fréquence augmente.

2. Appareil d'aide au sommeil (100) selon la revendication 1, dans lequel, dans ladite plage de fréquences prédéterminée, ladite intensité de signal est inversement proportionnelle à la fréquence.

3. Appareil d'aide au sommeil (100) selon la revendication 1, dans lequel, dans ladite plage de fréquences prédéterminée, ladite intensité de signal est inversement proportionnelle au carré de la fréquence.

4. Appareil d'aide au sommeil (100) selon l'une quelconque revendication précédente, dans lequel le volume dudit signal audio est limité à au moins l'un parmi 80 dBA d'émission moyenne dans le temps ou 110 dBC d'émission maximale, à une distance de 500 mm.

5. Appareil d'aide au sommeil (100) selon l'une quelconque revendication précédente, dans lequel ledit signal audio est atténué pour des fréquences supérieures à une fréquence prédéterminée, et dans lequel ledit signal audio est éventuellement atténué pour des fréquences supérieures à 1 kHz.

6. Appareil d'aide au sommeil (100) selon l'une quelconque revendication précédente, dans lequel ladite source sonore (120) est adaptée pour générer ledit signal audio pendant une première période de temps prédéterminée, et dans lequel ladite première période prédéterminée est éventuellement de 40 minutes.

7. Appareil d'aide au sommeil (100) selon l'une quelconque revendication précédente, dans lequel ledit signal audio est continu.

8. Appareil d'aide au sommeil (100) selon l'une quelconque revendication précédente, dans lequel l'appareil (100) comprend en outre un capteur audio pour détecter un signal audio externe, et dans lequel ledit signal audio est éventuellement généré en réponse au signal audio externe détecté.

9. Appareil d'aide au sommeil (100) selon l'une quelconque revendication précédente, comprenant en outre une autre source de lumière adaptée pour générer sélectivement un autre signal lumineux, projeté depuis ledit appareil d'aide au sommeil (100) avec une seconde luminosité et dans lequel ledit autre signal lumineux a une longueur d'onde choisie dans une plage de 600 nm à 700 nm, et dans lequel ladite seconde luminosité est éventuellement constante, et dans lequel l'appareil d'aide au sommeil (100) comprend éventuellement en outre un élément diffuseur (136) configuré pour diffuser uniformément l'autre signal lumineux.

10. Appareil d'aide au sommeil (100) selon l'une quelconque revendication précédente, dans lequel ladite première luminosité est sélectivement limitée à un maximum de 200 Lux.

11. Appareil d'aide au sommeil (100) selon l'une quelconque des revendications 9 à 10, dans lequel ladite seconde luminosité est sélectivement limitée à un maximum de 200 Lux.

12. Appareil d'aide au sommeil (100) selon l'une quelconque revendication précédente, dans lequel ladite première luminosité est périodiquement variable à un taux prédéterminé, et dans lequel ledit taux prédéterminé est éventuellement compris entre 6 fois par minute et 40 fois par minute, par exemple, ledit taux prédéterminé est éventuellement compris entre 8 fois par minute et 20 fois par minute.

13. Appareil d'aide au sommeil (100) selon la revendication 12, dans lequel ledit taux prédéterminé est configuré pour changer de façon incrémentielle au cours d'une seconde période de temps prédéterminée.

14. Appareil d'aide au sommeil (100) selon l'une quelconque revendication précédente, dans lequel ladite première luminosité varie périodiquement entre une luminosité maximale et une luminosité minimale, et dans lequel ladite luminosité minimale est égale à 5 % de ladite luminosité maximale.

15. Appareil d'aide au sommeil (100) selon l'une quelconque revendication précédente, dans lequel l'appareil (100) est configuré pour projeter le premier signal lumineux dans un faisceau étroit, et dans lequel le faisceau étroit se projette éventuellement depuis l'appareil (100) dans une configuration conique telle que le diamètre du faisceau étroit est de 30 cm à 60 cm à une distance de 140 cm de l'appareil (100).
